# FASCICULE DE BREVET EUROPEEN

(11) **EP 1 030 698 B1**
(45) Date de publication et mention de la délivrance du brevet: **28.01.2004**
(21) Numéro de dépôt: 99942991.3
(22) Date de dépôt: 16.09.1999
(51) Int. Cl.: A61L 31/00, A61L 15/32

(54) **MATERIAU COLLAGENIQUE BICOMPOSITE, SON PROCEDE D'OBTENTION ET SES APPLICATIONS THERAPEUTIQUES**
ZWEISCHICHTIGES KOLLAGENMATERIAL, DESSEN HERSTELLUNGSPROZESS UND THERAPEUTISCHE ANWENDUNG
BI-COMPOSITE COLLAGEN MATERIAL, METHOD FOR OBTAINING SAME AND THERAPEUTIC APPLICATIONS

(30) Priorité: 18.09.1998 FR 9811701
(43) Date de publication de la demande: 30.08.2000
(73) Titulaire: Imedex Biomateriaux, 69630 Chaponost (FR)
(72) Inventeur: BAYON, Yves, F-69100 Villeurbanne (FR); GRAVAGNA, Philippe, F-69540 Irigny (FR); TAYOT, Jean-louis, F-69890 La Tour de Salvagny (FR)
(74) Mandataire: Bernasconi, Jean Raymond
(86) Numéro de dépôt international: PCT/FR1999/002212
(87) Numéro de publication internationale: WO 2000/016821

(56) Documents cités:
- EP-A- 0 138 385
- EP-A- 0 664 132
- EP-A- 0 686 402
- WO-A-93/10731
- WO-A-96/08277
- WO-A-98/34656
- FR-A- 2 628 634
- US-A- 5 201 745

## Description

La présente invention a trait à un matériau bicomposite à base de collagène, biocompatible, non toxique et biodégradable, comprenant uniquement, ou principalement, une couche formant un film collagénique et une couche formant une compresse ou éponge de polymère fibreux de porosité élevée.

Le matériau selon l'invention peut être utilisé en chirurgie, notamment en chirurgie viscérale, et trouve une application particulière dans la réalisation simultanée de l'hémostase et de la prévention des adhérences post-opératoires tout en favorisant la cicatrisation du tissu lésé.

Les brevets FR-A-2 628 634 et US-A-5 201 745 (IMEDEX) décrivent des patchs de chirurgie viscérale réalisés à partir d'un biomatériau formé de deux couches de collagène superposées et associées intimement, à savoir une couche poreuse collable de collagène fibreux et un film de collagène ou de matériau collagénique tel que de la gélatine.

Dans un tel matériau, le film assure l'étanchéité de la membrane ou patch et augmente la cohésion mécanique et permet, en outre, de prévenir la formation des adhérences post-opératoires. La couche poreuse de collagène fibreux joue notamment un rôle de compresse hémostatique.

Une membrane collagénique bicouche a été proposée dans les demandes de brevet EP-A-0 686 402 et WO 96/08277 (COLETICA) en vue d'obtenir un pouvoir anti-adhérences.

Les collagènes et matériaux collagéniques utilisés dans de tels patchs ou membranes peuvent provenir du collagène natif ou de différents types d'atélocollagènes ou de collagènes pepsinés, et notamment de collagènes bovins de type I, de collagènes humains de type I, III, III + I, IV. Ces collagènes peuvent être partiellement oxydés, par exemple pour augmenter leur pouvoir adhérent, et la couche formant le film peut comporter, en mélange avec le matériau collagénique, d'autres matériaux permettant, par exemple, de renforcer ses propriétés de résistance mécanique et d'améliorer son pouvoir anti-adhérence.

La fabrication de ces patchs ou membranes n'est cependant pas aisée. En effet, il faut, d'une part assurer une excellente liaison entre la couche formant le film et la couche formant la compresse fibreuse, tout en individualisant bien les deux couches. En outre, lorsque l'on met en contact la couche de matériau fibreux avec le matériau collagénique liquide destiné à former le film, les fibres collagéniques, au contact du liquide, tendent à s'imprégner, de sorte que l'on peut effectivement obtenir une excellente liaison entre les deux couches mais avec une difficulté de bien contrôler la formation du film et de respecter la porosité de la couche support.

A cette fin, on a proposé (FR-A-2 628 634) de couler le matériau collagénique, devant former le film, sur une couche de collagène fibreux ayant subi préalablement un certain tassement pour limiter l'interpénétration entre les deux couches.

On a également déjà proposé (EP-A-0 686 402) de congeler la couche fibreuse poreuse pour lui conférer un état hydraté imperméable et de couler sur cette couche le matériau collagénique liquide destiné à former le film de façon à supprimer l'interpénétration entre les deux couches mais une telle suppression de l'interpénétration aboutit à un défaut de cohésion. Le procédé décrit aboutit en outre à une membrane bicouche collagène-gélatine ayant été séchée ou lyophilisée d'un seul tenant, ce qui empêche de former simultanément un film imperméable et une couche de porosité élevée. Il est en outre préconisé de compacter cette membrane.

Des éponges hémostatiques composées de collagène bovin natif sont commercialement disponibles, comme par exemples Colgen® (Immuno AG), Pangen® (Fournier), Surgicoll® (Biodynamics). Mais, elles ne sont pas recouvertes sur une face d'un film imperméable, jouant un rôle de barrière, et présentent plusieurs inconvénients :
i) laissées dans l'organisme, elles peuvent générer des adhérences ;
ii) le sang diffuse selon des parcours préférentiels dans la compresse, réduisant ainsi la surface de contact du collagène avec les plaquettes, et par la suite, le pouvoir hémostatique de la compresse ;
iii) elles ne jouent plus leur rôle hémostatique sur des plaies fortement hémorragiques (exemple rupture d'artérioles), le sang arrivant à passer à travers la compresse ;
iv) généralement produites à partir de collagène acide, elles sont difficiles à manipuler car elles collent fortement aux instruments de chirurgie ou aux gants en latex.

D'autres produits plus complexes comme le TachoComb® (Nycomed) associant le collagène, le fibrinogène, la thrombine et l'aprotinine permettent une hémostase plus efficace que les éponges de collagène. Mais, ces produits sont susceptibles de faciliter le développement d'adhérences post-opératoires. Comportant des enzymes thermolabiles, ils nécessitent un stockage entre 2 et 8°C. La multiplication des composants d'origine humaine ou animale est aussi handicapante, à cause des problèmes de traçabilité et d'enregistrements liés à ces produits, qui entraînent un surcoût prohibitif.

Du point de vue de la prévention des adhérences post-opératoires, celle-ci est particulièrement difficile sur des plaies hémorragiques et, plus particulièrement, sur des plaies saignant de façon très diffuse (Buckman et al., J. Surg. Res., 1976, 20, 1-5 ; Wiseman et al., J. Reprod. Med., 1992, 37, 766-770). Le saignement de plaies compromet fortement l'efficacité de produits commercialisés et utilisés pour la prévention des adhérences comme l'INTERCEED® TC7 (Johnson & Johnson) (Wiseman et al., J. Reprod. Med., 1992, 37, 766-770). En effet, il peut entraîner le dépôt de fibrine sur les films anti-adhérents et faciliter, par suite, le développement d'adhérences post-opératoires. Il en résulte qu'il faut procéder à l'hémostase la plus complète possible, à l'aide de thrombine ou de toutes autres techniques, avant d'appliquer sur les plaies hémorragiques des produits comme l'INTERCEED® TC7. Il est donc intéressant pour la prévention des adhérences de développer des matériaux présentant également un pouvoir hémostatique.

La présente invention a donc pour but de perfectionner considérablement les matériaux collagéniques bicomposites antérieurs décrits précédemment et d'accroître de manière importante leurs propriétés hémostatiques, tout en conservant et, le cas échéant, même en améliorant les propriétés d'anti-adhérences post-opératoires.

L'invention a également pour objectif de fournir un matériau bicomposite collagénique hémostatique, capable en plus de prévenir les adhérences post-opératoires et de faciliter la cicatrisation.

Un autre but de l'invention est de réaliser un tel matériau qui soit particulièrement bien colonisable par les cellules spécifiques de l'organisme et susceptible d'être biodégradé intégralement dans un délai réduit et facile à contrôler par de simples modifications du procédé de fabrication.

L'invention a également pour objectif de fournir un matériau bicomposite biocompatible, non toxique et non collant au toucher à l'état sec pour la facilité de manipulation, mais pouvant développer des propriétés adhésives en milieu physiologique, en particulier au contact du sang.

Un autre objectif de l'invention est de fournir un procédé particulièrement économique pour l'obtention d'un tel matériau bicomposite.

L'invention a ainsi pour objet un matériau collagénique bicomposite qui est biocompatible, non toxique et biodégradable en moins d'un mois, caractérisé en ce qu'il comprend uniquement, ou principalement, deux couches intimement liées, à savoir une couche formant un film à base d'un composant collagénique, notamment de collagène ayant perdu au moins partiellement sa structure hélicoïdale, ou de gélatine, et une couche formant une compresse poreuse sensiblement non tassée à base d'un composant polymère.

De préférence, le film comprend, outre le composant collagénique, au moins un additif hydrophile macromoléculaire chimiquement non réactif vis-à-vis du collagène.

La seconde couche peut être faite d'une compresse poreuse sensiblement non tassée de collagène non dénaturé.

L'invention a également pour objet un procédé préféré pour la réalisation de tels matériaux.

Ce procédé repose sur la découverte que, lorsqu'on laisse se gélifier une solution liquide à base de composant collagénique destinée à former un film, il existe un moment, au cours de la gélification, pendant lequel on peut poser la couche poreuse de composant polymère formant la compresse sur la surface du matériau en cours de gélification, et où la partie inférieure de ladite couche poreuse pénètre partiellement dans le gel, tout en conservant au moins partiellement une structure assurant une liaison pratiquement parfaite entre le film qui va être constitué et la couche poreuse, et en laissant pratiquement intégralement conservées les propriétés séparées de la couche poreuse et du film.

Les inventeurs ont constaté, de manière tout à fait surprenante, que :
- le film de collagène peut se former par déshydratation de la couche liquide de collagène malgré la présence d'une couche poreuse lyophilisée par dessus ;
- la couche poreuse supérieure n'est pas altérée ou modifiée lors de l'association avec le film en formation.

L'invention a donc pour objet un procédé pour l'obtention d'un matériau bicomposite selon l'invention, caractérisé en ce que l'on coule, sur un support inerte convenable, une solution de composant collagénique suivant une épaisseur destinée à la formation d'un film, et en ce que l'on applique sur ladite solution en cours de gélification, une compresse sensiblement non tassée de composant polymère, puis en ce que l'on sèche ou laisse sécher le matériau obtenu.

Le procédé selon l'invention va être décrit plus en détails ci-après :

Pour la mise en oeuvre de ce procédé, on prépare une solution aqueuse de composant collagénique destinée à la formation du film du matériau bicomposite précité.

Selon l'invention, le terme «composant collagénique» désigne de préférence du collagène ayant perdu au moins partiellement sa structure hélicoïdale par une opération de chauffage ou toute autre méthode, ou de la gélatine.

Le terme «gélatine» englobe ici la gélatine du commerce formée de collagène dénaturé par chauffage dont les chaînes sont, au moins partiellement, hydrolysées (poids moléculaires inférieurs à 100 kDa).

Le composant collagénique utilisé aux fins de l'invention est de préférence formé de collagène non hydrolysé, constitué majoritairement de chaînes α (poids moléculaire d'environ 100 kDa).

Par chaînes α au sens de l'invention, on entend des chaînes α entières ou des fragments issus de ces chaînes α entières par la perte d'un petit nombre d'acides aminés.

Le terme «non hydrolysé» tel qu'employé selon l'invention signifie que moins de 10 % des chaînes collagéniques ont un poids moléculaire inférieur à environ 100 kDa.

Dans le cas d'un chauffage pour dénaturer la structure hélicoïdale du collagène, le chauffage doit être modéré et effectué dans des conditions douces, de manière à éviter la dégradation par coupure hydrolytique de la gélatine ainsi formée.

La gélatine du commerce est utilisable aux fins de l'invention mais n'est pas préférée.

Le collagène mis en oeuvre est indifféremment d'origine humaine ou animale. Il peut s'agir en particulier de collagène bovin de type I ou de collagènes humains de type I ou de type III ou encore de mélanges en toutes proportions de ces derniers.

On utilise de préférence, du collagène natif en solution acide ou après traitement, pour éliminer les télopeptides, notamment par digestion à la pepsine.

Par ailleurs, le collagène peut être modifié par coupure oxydative. On peut utiliser à cet effet l'acide periodique ou l'un de ses sels selon la technique décrite par M. TARDY et coll. (FR-A-2 601 371 et US-A-4 931 546).

On rappelle brièvement que cette technique consiste à mélanger le collagène en solution acide, avec une solution d'acide periodique ou l'un de ses sel à une concentration comprise entre 1 et 10⁻⁵ M, de préférence entre 5 10⁻³ et 10⁻¹ M, à une température comprise entre 10 et 25°C pendant une durée pouvant aller de 10 minutes à 72 heures.

Ce traitement provoque des coupures dans certains composants du collagène qui sont l'hydroxylysine et les sucres, et crée ainsi des sites réactifs sans en provoquer la réticulation.

La coupure oxydative du collagène a comme fonction de permettre une réticulation modérée ultérieure du matériau collagénique mais l'invention n'exclut pas que l'on puisse réaliser cette fonction par d'autres moyens de réticulation modérée, par exemple par rayonnement béta ou gamma, ou d'autres agents de réticulation modérée, par exemple des agents chimiques à des doses suffisamment faibles et non toxiques.

Pour certaines applications, on emploie pour le film du matériau bicomposite selon l'invention, du collagène qui n'est pas oxydé ou un mélange, en toutes proportions, de collagène non oxydé et de collagène oxydé.

Dans un mode de mise en oeuvre préféré de l'invention, on utilise une solution de composant collagénique tel que défini ci-dessus, éventuellement partiellement ou totalement modifié par coupure oxydative, dont la concentration en collagène est comprise entre 5 et 50 g/l. La concentration en collagène ou gélatine est de préférence de 30 g/l.

La solution de collagène oxydé, de collagène non oxydé ou de leurs mélanges, ainsi préparée est chauffée, par exemple à une température supérieure à 37°C, de préférence à une température comprise entre 40 et 50°C, pendant moins d'une heure. Il en résulte que la structure hélicoïdale du collagène est dénaturée, au moins partiellement.

On peut obtenir notamment une préparation finale que l'on peut assimiler à de la gélatine mais dont le poids moléculaire des chaînes élémentaires est supérieur ou égal à 100 kDa.

Le traitement par chauffage à température supérieure à 37°C de la solution de collagène entraîne la perte progressive de la structure hélicoïdale du collagène, mais l'invention n'exclut pas que l'on puisse réaliser cette fonction par d'autres moyens physiques ou chimiques, par exemple par ultra-sonication, ou par addition d'agents chaotropiques.

Selon une variante de réalisation de l'invention, on ajoute à la préparation précédente, au moins un additif hydrophile macromoléculaire, de préférence chimiquement non réactif vis-à-vis du composant collagénique

Par «chimiquement non réactif vis-à-vis du composant collagénique», on entend un composé hydrophile qui n'est pas susceptible de réagir avec le collagène présent, notamment qui ne forme pas de liaison covalente avec celui-ci lors de sa réticulation.

L'additif hydrophile macromoléculaire selon l'invention présente un poids moléculaire avantageusement supérieur à 3 000 Daltons.

Il peut s'agir de polymères hydrophiles de synthèse avantageusement de poids moléculaire compris entre 3 000 et 20 000 Daltons. Le polyéthylèneglycol est particulièrement préféré.

Il peut s'agir également de polysaccharides parmi lesquels on peut citer l'amidon, le dextrane et la cellulose.

On peut également prévoir l'utilisation de tels polysaccharides sous forme oxydée faisant apparaître des fonctions carboxyliques dans ces molécules.

Les mucopolysaccharides peuvent également convenir aux fins de l'invention mais ne sont pas préférés car leur origine animale particulière les rend difficiles à préparer en satisfaisant aux normes réglementaires de traçabilité.

L'additif hydrophile est sélectionné en fonction de divers paramètres liés notamment à son application, comme son prix, son innocuité, sa biodégradabilité et/ou sa facilité d'élimination.

La concentration en additif(s) hydrophile(s) est de 2 à 10 fois inférieure à celle du composant collagénique.

Selon une variante de réalisation de l'invention, on prévoit d'ajouter au mélange composant collagénique/additif(s) hydrophile(s), de la glycérine.

Dans ce cas, la concentration en glycérine est avantageusement comprise entre 3 et 8 g/l, ne dépassant pas le tiers de la concentration en composant collagénique.

Dans la préparation collagénique, les concentrations en composant collagénique, en additif(s) hydrophile(s) et en glycérine lorsqu'ils sont présents, sont de préférence comprises entre 2 et 10 % pour le composant collagénique, entre 0,6 et 4 % pour le (les) additif(s) hydrophile(s) et entre 0,3 et 2,5 % pour la glycérine respectivement.

La préparation collagénique est fluidifiée à une température comprise entre 30 et 50°C.

Elle est avantageusement neutralisée jusqu'à pH neutre pour éviter l'hydrolyse du collagène par chauffage et en vue d'obtenir un film à pH physiologique tout en permettant la pré-réticulation du collagène si le mélange contient du collagène oxydé comme indiqué précédemment.

Pour la mise en oeuvre du procédé selon l'invention, on prépare par ailleurs une compresse poreuse sensiblement non tassée à base de composant polymère.

Par «composant polymère», on entend selon l'invention un polymère fibreux, non toxique présentant des propriétés hémostatiques et/ou cicatrisantes. Il peut s'agir de collagène non dénaturé ou de collagène ayant perdu au moins partiellement sa structure hélicoîdale par une opération de chauffage ou toute autre méthode, constitué majoritairement de chaînes α non hydrolysées, de poids moléculaire voisin de 100 kDa. Il peut s'agir aussi de polysaccharides tels que la chitine ou le chitosane, ou de polysaccharides modifiés par oxydation des fonctions alcools en fonctions carboxyliques tels que la cellulose oxydée.

Par «collagène non dénaturé» on entend du collagène qui n'a pas perdu sa structure hélicoïdale.

Le collagène utilisé pour cette seconde couche du matériau bicomposité selon l'invention, consiste en du collagène natif ou en de l'atélocollagène, notamment tel qu'obtenu par digestion à la pepsine ou/et après chauffage modéré tel que défini auparavant.

Ceux-ci peuvent avoir été préalablement modifiés chimiquement par oxydation, méthylation, succinylation ou tout autre procédé connu en soi.

L'origine et le type de collagène sont comme indiqué dans le cas du film décrit ci-dessus.

Définie sous une autre forme, ladite couche formant compresse poreuse présente une densité inférieure ou égale à 75 mg/cm² et de préférence inférieure à 20 mg/cm².

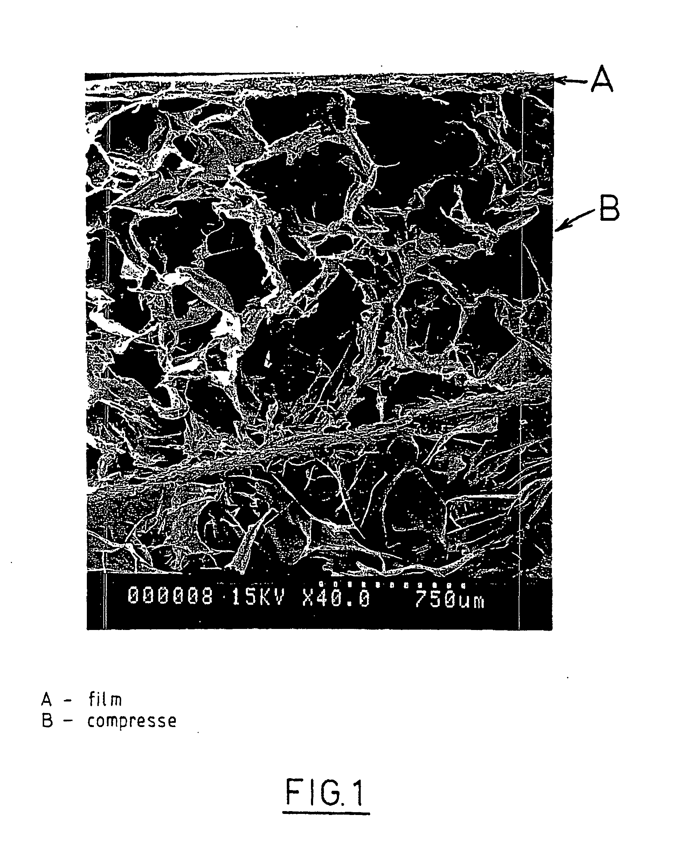

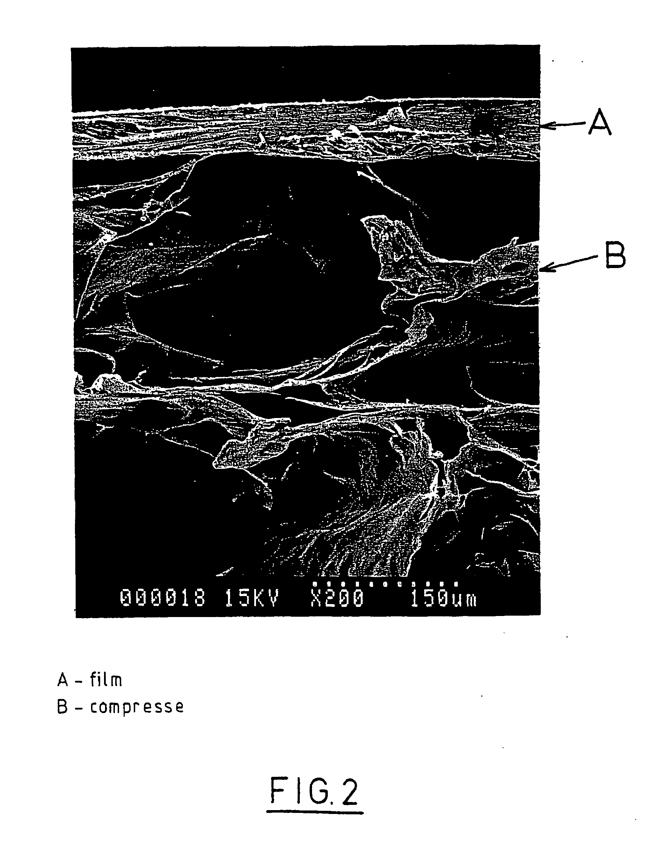

La porosité de ces matériaux est illustrée aux figures 1 et 2.

La taille des pores varie de 20 à 300 µm et est en moyenne comprise entre 100 et 200 µm.

La compresse poreuse peut être obtenue, de préférence par lyophilisation, à partir d'une solution aqueuse acide de collagène dont la concentration est comprise entre 2 et 50 g/l et dont la température est comprise entre 4 et 25°C. La concentration en collagène est de préférence de 10 g/l.

Cette solution est avantageusement neutralisée de préférence jusqu'à un pH de l'ordre de 7 à 8.

La compresse poreuse peut être obtenue aussi par lyophilisation d'une mousse fluide préparée à partir d'une solution de collagène ou de collagène chauffé, émulsionnée en présence d'un volume d'air en quantités respectives variables (volume air : eau variant de 1 à 10).

L'épaisseur de la couche fibreuse poreuse de composant polymère est de préférence au moins égale à 0,2 cm, et de façon particulièrement préférée comprise entre 0,3 et 1,5 cm.

La préparation du matériau bicomposite proprement dite est réalisée par l'assemblage de la couche formant film et de la compresse poreuse, comme indiqué ci-après.

Dans son mode de mise en oeuvre le plus simple, le procédé selon l'invention comporte le coulage de la solution de composant collagénique, destinée à réaliser le film, contenant éventuellement le (les) additif(s) hydrophile(s) et la glycérine, sur un support sensiblement plan adéquat, en répartissant celle-ci uniformément.

Le support est inerte en ce qu'il ne réagit pas avec les composants précités et n'intervient pas dans le processus de réticulation. Il est de préférence hydrophobe, par exemple en PVC ou polystyrène.

Cependant, ce support peut également être constitué d'un matériau pelliculable qui restera faiblement adhérent et qui pourra ensuite être séparé au moment de l'utilisation chirurgicale.

Ce support peut encore être lui-même constitué d'un film, par exemple de collagène séché, sur lequel on coule la solution, ou encore d'une couche de gel de matériau collagénique à un état de gélification nettement plus avancé.

La densité de la couche mince appliquée est de préférence comprise entre 0,1 et 0,3 g/cm².

Le coulage de cette solution collagénique est réalisé à une température avantageusement comprise entre 4 et 30°C, de préférence entre 18 et 25°C.

On laisse cette solution se gélifier et on applique sur ladite solution en cours de gélification, une compresse poreuse préparée comme indiqué précédemment.

Par application de la couche poreuse sur la solution en cours de gélification, on entend la dépose de la couche poreuse sur le gel, l'application se poursuivant par simple gravité ou, éventuellement par une légère compression insuffisante pour provoquer un tassement sensible de la couche poreuse.

Le moment auquel on applique la couche poreuse sur la solution en cours de gélification est tel que le gel est encore mou et laisse pénétrer la couche poreuse ou compresse sur une distance qui est avantageusement de l'ordre de 0,05 à 2 mm, de préférence de l'ordre de 0,1 à 0,5 mm.

Ce moment peut être déterminé de façon empirique en appliquant des compresses ou morceaux de compresse sur le gel à des moments différents.

En général, lorsque la solution qui se gélifie est à une température comprise entre 4 et 30°C, la couche poreuse est appliquée entre 5 et 30 minutes après la répartition de la solution sur la surface qui la reçoit.

On laisse sécher ou on sèche l'ensemble pour obtenir le matériau bicomposite selon l'invention.

Lorsque la solution collagénique destinée à la formation du film comprend du collagène oxydé, sa polymérisation s'effectue pendant le séchage du matériau bicomposite.

Ce séchage est avantageusement obtenu à une température comprise entre 4 et 30°C, de préférence entre 18 et 25°C.

On peut réaliser le séchage du matériau dans un flux d'air stérile, si nécessaire.

Après séchage, le matériau bicomposite selon l'invention peut être séparé de son support. En variante, il peut comprendre ou incorporer un film ou une couche de matériau collagénique sur lequel la solution collagénique a été coulée.

Le procédé décrit ci-dessus peut être mis en oeuvre d'une manière similaire avec d'autres types de compresses hémostatiques, notamment des compresses telles que disponibles dans le commerce. On peut citer par exemples des compresses à base de cellulose oxydée (compresses Surgicel® ou Interceed®) ou à base de chitine ou chitosane.

Le matériau bicomposite selon l'invention est stable à température ambiante et reste stable pendant un temps suffisant pour sa manipulation à des températures pouvant aller jusqu'à 37-40°C.

L'épaisseur du film de matériau collagénique est de préférence inférieure à 100 µm, et plus préférentiellement comprise entre 30 et 75 µm.

L'épaisseur de la compresse poreuse est de préférence comprise entre 0,2 cm et 1,5 cm, plus préférentiellement encore entre 0,3 cm et 1,2 cm.

Selon les applications envisagées, le matériau bicomposite conforme à l'invention peut être soumis à divers traitements classiques tels que stérilisation, etc.

La stérilisation est avantageusement opérée par irradiation avec des rayonnements béta (irradiation électronique) ou gamma (irradiation par le cobalt radioactif).

Le matériau bicomposite selon l'invention peut être utilisé tel quel ou découpé aux dimensions appropriées à l'application envisagée.

La présente invention a permis de réaliser des matériaux bicomposites dans lesquels une couche de polymère fibreux, notamment de collagène non dénaturé, extrêmement poreuse et pouvant avoir une grande épaisseur, pour former une compresse ou éponge efficace, est très étroitement liée à un film collagénique mince, bien délimité et de propriétés et de dimensions convenables.

Il a ensuite pu être établi qu'un tel matériau bicouche présentait un ensemble de qualités d'hémostase, d'anti-adhérences post-opératoires et de bio-dégradabilité particulièrement surprenantes.

Le biomatériau obtenu est de manipulation aisée. Il ne colle pas aux instruments chirurgicaux ou aux gants, à l'état sec.

Il présente une résistance mécanique acceptable tout en gardant une certaine souplesse, apportée par les éléments hydrophiles contenus dans le film du matériau composite.

Le matériau selon l'invention est un hémostatique local dont le principe actif est le composant polymère, notamment le collagène non dénaturé ou la cellulose oxydée, qui participe, au même titre que le collagène endogène, au processus de l'hémostase et de la cicatrisation. Il est appliqué de préférence avec pression sur le site hémorragique jusqu'à l'obtention d'une hémostase. Le sang est absorbé par la couche poreuse du matériau et concentré sous le matériau, grâce au film du matériau, jouant le rôle de barrière étanche. Au contact du polymère, il est transformé en clou hémostatique et/ou en caillot.

Très rapidement, le matériau adhère à la plaie hémorragique, grâce à la formation du clou hémostatique et/ou du caillot par le polymère.

On pense que les propriétés hémostatiques considérablement accrues de la compresse selon l'invention sont dues notamment à la possibilité d'absorber une quantité très importante de sang tout en empêchant sa diffusion aussi bien transversalement au biomatériau que dans son plan. En outre, la diffusion du sang dans la compresse poreuse, dans la zone délimitée par la plaie, accroît la surface de contact entre la substance hémostatique et les plaquettes. Elle accélère ainsi l'arrêt du saignement en jouant sur les voies diverses de la coagulation, dont la phase finale conduit à la formation d'un réseau de plaquettes et de fibrine renforçant l'adhésion de la compresse sur la plaie.

Au contraire, les matériaux collagéniques bicouches de l'art antérieur décrit ci-dessus présentent une porosité insuffisante qui ne permet pas la pénétration du sang. Ceci favorise des fuites latérales de sang sous la compresse ce qui ne permet pas une bonne adhésion. De ce fait, l'arrêt du saignement est beaucoup plus difficile.

Le matériau collagénique bicomposite selon l'invention est particulièrement adapté à la prévention d'adhérences post-opératoires, en particulier sur des plaies hémorragiques, du fait de la prévention des adhérences par le film, de la bonne adhésion du matériau composite sur de telles plaies et de l'absence de sang à l'interface.

Outre leurs propriétés d'hémostase et de prévention d'adhérences post-opératoires, le matériau collagénique relevant de la présente invention facilite la cicatrisation, à cause de sa structure composite, associant une couche très poreuse de polymère à un film collagénique.

La partie poreuse du matériau est facilement colonisable par les cellules environnantes. Le film protège la cicatrisation en cours pendant quelques jours grâce à ses propriétés d'étanchéité aux bactéries et microorganismes.

Le pouvoir de prévention des adhérences par le film du matériau est également renforcé par l'accélération de la cicatrisation de la plaie par le polymère de la couche poreuse du matériau.

Selon l'invention, le matériau collagénique bicomposite est ainsi utile pour l'hémostase et la prévention des adhérences post-opératoires sur des plaies saignantes tout en facilitant la cicatrisation.

En outre, l'additif hydrophile macromoléculaire est éliminé par diffusion à travers le matériau collagénique, en quelques jours, matériau dont le gonflement favorise la dégradation du film collagénique en moins d'un mois.

Le matériau bicomposite selon l'invention peut également être utilisé pour favoriser la cicatrisation. Sa structure poreuse très ouverte permet une colonisation cellulaire rapide. Le film permet, quant à lui, d'isoler la partie poreuse pour la rendre accessible à des cellules spécifiques.

A titre d'exemple, des fibroblastes peuvent être cultivés dans la partie poreuse du matériau, in vitro, et des cellules épithéliales peuvent être cultivées sur le film en réalisant deux compartiments provisoirement séparés.

Cependant on peut également, bien que cela ne soit pas préféré, relier un film de composant collagénique et une compresse poreuse non tassée par l'intermédiaire d'un agent adhésif biocompatible, biodégradable et non toxique, dès lors que cet agent peut assurer une liaison suffisamment forte entre le film et la compresse, tout en n'étant présent qu'en faible quantité.

Des exemples d'agent adhésif sont les colles chirurgicales, notamment les colles de fibrine et les colles collagéniques décrites dans le brevet Tardy et al. US-A-5 618 551 et la demande WO 98/15299.

Cette invention va être maintenant décrite en détail à l'aide d'exemples non limitatifs montrant différentes compositions possibles du matériau ainsi que leurs pouvoirs hémostatiques et leurs capacités de prévention des adhérences tissulaires post-opératoires.

Selon l'invention, on peut soit réaliser une compresse qui sera redécoupée aux dimensions et à la forme voulues ou préparer un biomatériau ayant les dimensions et les formes du patch désiré.

### EXEMPLES

### Exemple 1 :

### Préparation de compresses de collagène à pH neutre :

Le collagène utilisé est du collagène bovin de type I, extrait de derme de veau, éventuellement solubilisé par digestion à la pepsine et, purifié par précipitations salines, selon les techniques déjà décrites. On peut utiliser, de la même manière, des collagènes humains de type I ou III ou leur mélange en toutes proportions.

Une solution de collagène à 10 g/l est préparée par dissolution de 23 g de collagène humide (taux d'humidité de 12 %) dans 2070 g d'eau ultrafiltrée, à la température ambiante restant inférieure à 25°C. Elle est neutralisée à l'aide d'hydroxyde de sodium jusqu'à pH neutre, ce qui entraîne une précipitation du collagène.

La suspension est, ensuite, répartie dans des plateaux de lyophilisation, à raison de 0,5 à 1 g/cm² et déshydratée par lyophilisation, en prenant un cycle durant environ 24 heures.

En final la compresse lyophilisée de collagène peut être soumise dans une variante à un chauffage à 60°C pendant plusieurs heures (4 à 15) qui permet une cohésion et une résistance mécanique supérieures dans certaines applications.

### Exemple 2 :

### Préparation de compresses de collagène à pH 5-5,5 :

La préparation de compresses de collagène à pH 5-5,5 permet de limiter le phénomène de précipitation du collagène. Elle est réalisée suivant l'exemple 1, avec comme seule différence la neutralisation de la solution de collagène à l'aide d'hydroxyde de sodium à un pH voisin du point isoélectrique du collagène, c'est-à-dire 5 et 5,5.

### Exemple 3 :

### Préparation de compresses de collagène à pH acide :

La préparation de compresses légèrement acides est réalisée suivant l'exemple 1, avec comme seule différence que la neutralisation de la solution de collagène n'est pas effectuée, ce qui évite toute précipitation du collagène.

### Exemple 4 :

### Préparation d'une solution de collagène oxydé :

Le collagène oxydé à 30 g/l, utilisé pour cet exemple, est préparé suivant le brevet FR-A-2 715 309. On emploie du collagène bovin de type I, extrait de derme de veau par solubilisation à pH acide ou par digestion à la pepsine et purifié par précipitations salines selon des techniques déjà décrites.

Les produits commercialisés par la société COLLAGEN Corp., sous les noms de VITROGEN® ou ZYDERM®, peuvent convenir dans cette application.

On utilise de préférence des fibres sèches de collagène, obtenues par précipitation d'une solution acide de collagène par addition de NaCl, puis lavages et séchage du précipité obtenu par des solutions aqueuses d'acétone de concentration croissante de 80 % à 100 %.

On peut, de la même manière, utiliser des collagènes humains de type I ou III ou leur mélange en toutes proportions.

La solution de collagène à 30 g/l est préparée par dissolution en HCl 0,01 N. Son volume est de 49 litres. Elle est additionnée d'acide periodique à une concentration finale de 8 mM soit 1,83 g/l.

On réalise l'oxydation à température ambiante voisine de 22°C, pendant 3 heures à l'abri de la lumière.

Puis, la solution est additionnée d'un volume égal d'une solution de chlorure de sodium pour obtenir une concentration finale de 41 g/l NaCl.

Après 30 minutes d'attente, le précipité est récolté par décantation à travers un tamis de toile de porosité voisine de 100 microns, puis lavé 4 fois avec une solution de NaCl 41 g/l en HCl 0,01 N. On obtient 19 kg de précipité salin acide. Ces lavages permettent l'élimination de toutes traces d'acide periodique ou dérivés iodés formés pendant l'oxydation du collagène.

Ensuite, plusieurs lavages en solution aqueuse d'acétone à 80 % permettent la concentration du précipité de collagène et l'élimination des sels présents.

Un lavage final en acétone 100 % permet de préparer 3,6 kg d'un précipité acétonique très dense, de collagène oxydé acide, non réticulé, ne contenant aucune trace de produit chimique indésirable.

La pâte acétonique est reprise en eau distillée apyrogène à 40°C, pour obtenir une concentration de collagène de 3 %, d'un volume de 44 litres. Cette suspension de collagène oxydé permet de préparer des compresses poreuses de manière semblable aux exemples 1, 2 et 3.

### Exemple 5 :

### Préparation d'une solution de collagène chauffé :

Un gel de collagène à pH neutre de concentration voisine de 50 g/l est chauffé à 45°C pendant 10 minutes pour le fluidifier.

4 volumes d'air ou autre gaz sont incorporés dans la solution de collagène chauffé à l'aide de 2 seringues montées face à face et connectées pour réaliser l'émulsion, grâce à des allers retours successifs des pistons de seringues, qui mélangent les contenus respectifs de chaque seringue de manière homogène.

L'émulsion est conditionnée en plateaux de lyophilisation et gélifiée par refroidissement, puis congelée et lyophilisée.

### Exemple 6 :

### Préparation d'une solution de collagène oxydé chauffé destiné à la formation du film.

La suspension décrite à l'exemple 4, d'un volume de 44 litres est chauffée 30 minutes à 50°C, puis filtrée stérilement sur membrane de porosité 0,45 microns dans une étuve à 40°C.

Dès que cette solution est homogène et à 35°C, elle est additionnée d'une solution stérile concentrée de PEG 4000 (polyéthylèneglycol de poids moléculaire 4000 Daltons) et de glycérine pour obtenir une concentration finale de 0,9 % en PEG, de 0,54 % en glycérine et de 2,7 % en collagène oxydé.

Dès la fin de ces additions, le pH de la solution est ajusté à 7,0, par addition d'une solution concentrée d'hydroxyde de sodium.

### Exemple 7 :

### Préparation d'une solution comportant un mélange de collagène non oxydé chauffé et de collagène oxydé, destinée à la formation du film :

Une variante de préparation de la solution de collagène, utilisée pour le film, est de prendre du collagène non oxydé chauffé ou un mélange de collagène oxydé chauffé, préparé suivant l'exemple 6, et de collagène non oxydé chauffé, en toutes proportions.

Le collagène utilisé pour la préparation du collagène non oxydé chauffé est du collagène bovin de type I, extrait de derme de veau, éventuellement solubilisé par digestion à la pepsine et, purifié par précipitations salines, selon les techniques déjà décrites. On peut utiliser, de la même manière, des collagènes humains de type I ou III ou leur mélange en toutes proportions.

Une solution de collagène non oxydé chauffé à 30 g/l est préparée par dissolution de 65,2 g de collagène humide (taux d'humidité de 12 %) dans 1940 g d'eau ultrafiltrée, à 42°C. Cette solution est additionnée à 42°C d'une solution stérile concentrée de PEG 4000 (polyéthylèneglycol de poids moléculaire 4000 Daltons), de glycérine et, éventuellement de collagène oxydé chauffé, préparé suivant l'exemple 6, pour obtenir une concentration finale de 0,9 % en PEG, de 0,54 % en glycérine et de 2,7 % en collagène total. Le pH de la solution est ajusté à 7,0, par addition d'une solution concentrée d'hydroxyde de sodium.

### Exemple 8 :

### Préparation d'une solution acide de collagène non oxydé chauffé, destinée à la formation du film :

La préparation d'une solution acide de collagène non oxydé chauffé, pour le film, est réalisée suivant l'exemple 7, avec les différences suivantes :
i) le collagène utilisé est exclusivement du collagène non oxydé chauffé dont la préparation est décrite dans l'exemple 1 ;
ii) le mélange utilisé pour le film, dont les concentrations finales en PEG, glycérine et collagène sont respectivement de 0,9 %, 0,54 % et 2,7 %, est acide.

### Exemple 9 :

### Préparation d'un matériau bicomposite à partir d'une compresse collagénique :

La solution de collagène, destinée à la formation du film, comme décrite dans les exemples 4 à 7, est coulée en couche mince à densité de 0,133 g/cm² sur un support plan hydrophobe de type PVC ou polystyrène, à température ambiante, voisine de 22°C.

Une compresse de collagène, préparée suivant les exemples 1, 2 ou 3 est appliquée uniformément sur la solution de collagène chauffé, 5 à 20 minutes après sa répartition sur le support. Ce temps d'attente correspond au temps de gélification de la solution de collagène, nécessaire à l'application de la compresse de collagène, pour éviter sa dissolution ou son hydratation partielle dans le collagène liquide.

La pénétration de la compresse dans la solution gélifiée de collagène est jugée inférieure à 0,5 mm.

L'ensemble est ensuite déshydraté sous un flux d'air stérile, à température ambiante, qui conduit à l'évaporation en 18 heures environ.

Le matériau bicomposite obtenu se décolle très facilement du support.

Il peut être découpé, sans le fragiliser, aux dimensions souhaitées pour l'application recherchée.

Ensuite, le matériau bicomposite est inclus dans un double sachet en polyéthylène, non perméable à l'air.

L'ensemble est stérilisé par irradiation gamma ou par faisceau d'électrons (irradiation béta) à une dose comprise entre 25 et 35 KGy.

Le matériau est stable à température ambiante.

La présence de glycérine dans le matériau contribue essentiellement à conférer une plus grande souplesse au film et en facilite l'utilisation. Le matériau peut ne pas contenir de glycérine.

L'utilisation de PEG 4000 comme agent hydrophile macromoléculaire n'est pas limitatif. On peut lui substituer du PEG 3000, PEG 6000 ou des polysaccharides comme l'amidon soluble (OSI, France) et le Dextran T40 (Pharmacia Fine Chemicals, Suède).

Les figures 1 et 2 sont des photographies prises au microscope électronique à balayage, respectivement à un grossissement de 40 et de 200, qui illustrent les structures du matériau bicomposite préparés comme indiqué ci-dessus.

La figure 1 correspond à l'exemple 9 mis en oeuvre à partir de la compresse de l'exemple 1 préparée à partir du collagène pepsiné, le film étant réalisé selon l'exemple 6.

La figure 2 correspond à l'exemple 9 mis en oeuvre à partir de la compresse de l'exemple 3, le film étant réalisé selon l'exemple 8.

### Exemple 10 :

### Préparation d'un matériau bicomposite à partir d'une compresse de cellulose oxydée :

On procède comme à l'exemple 9 mais en utilisant une compresse poreuse à base de cellulose oxydée telle que disponible dans le commerce sous les noms de Interceed® ou Surgicel®.

## Revendications

1. Matériau collagénique bicomposite qui est biocompatible, non-toxique et biodégradable en moins d'un mois, **caractérisé en ce qu'**il comprend uniquement, ou principalement, deux couches intimement liées, à savoir une couche formant un film à base d'un composant collagénique, notamment de collagène ayant perdu au moins partiellement sa structure hélicoïdale, ou de gélatine, et une couche formant une compresse poreuse non tassée à base d'un composant polymère.

2. Matériau collagénique bicomposite selon la revendication 1, **caractérisé en ce que** le composant collagénique consiste en ou comprend du collagène ayant perdu au moins partiellement sa structure hélicoïdale.

3. Matériau collagénique bicomposite selon la revendication 2, **caractérisé en ce que** le composant collagénique est formé de collagène non hydrolysé, constitué majoritairement de chaînes α.

4. Matériau collagénique bicomposite selon l'une quelconque des revendications 1 à 3, **caractérisé en ce que** le composant collagénique consiste en ou comprend de la gélatine.

5. Matériau collagénique selon l'une quelconque des revendications 1 à 4, **caractérisé en ce que** le composant collagénique consiste en ou comprend du collagène modifié par coupure oxydative.

6. Matériau collagénique bicomposite selon l'une quelconque des revendications 1 à 5, **caractérisé en ce que** la couche formant film comprend en outre au moins un additif hydrophile macromoléculaire chimiquement non réactif vis-à-vis du composant collagénique.

7. Matériau collagénique selon la revendication 6, **caractérisé en ce que** l'additif hydrophile macromoléculaire présente un poids moléculaire supérieur à 3000 Daltons.

8. Matériau collagénique bicomposite selon la revendication 7, **caractérisé en ce que** l'additif hydrophile macromoléculaire présente un poids moléculaire compris entre 3 000 et 20 000 Daltons.

9. Matériau collagénique bicomposite selon l'une quelconque des revendications 6 à 8, **caractérisé en ce que** l'additif hydrophile macromoléculaire est du polyéthylèneglycol.

10. Matériau collagénique bicomposite selon l'une quelconque des revendications 6 à 9, **caractérisé en ce que** l'additif hydrophile est choisi parmi les polysaccharides, notamment l'amidon, le dextrane ou la cellulose, ou les mucopolysaccharides.

11. Matériau collagénique bicomposite selon l'une quelconque des revendications 6 à 10, **caractérisé en ce que** l'additif hydrophile est un polysaccharide sous forme oxydée.

12. Matériau collagénique bicomposite selon l'une quelconque des revendications 6 à 11, **caractérisé en ce que** la concentration en additif(s) hydrophile(s) est 2 à 10 fois inférieure à celle du composant collagénique.

13. Matériau collagénique bicomposite selon l'une quelconque des revendications 6 à 12, **caractérisé en ce que** la couche formant film comprend en outre de la glycérine.

14. Matériau collagénique bicomposite selon l'une quelconque des revendications 1 à 13, **caractérisé en ce que** la couche formant film présente une épaisseur inférieure à 100 µm, de préférence comprise entre 30 µm et 75 µm.

15. Matériau collagénique bicomposite selon l'une quelconque des revendications 1 à 14, **caractérisé en ce que** le composant polymère de la couche poreuse formant compresse consiste en ou comprend du collagène.

16. Matériau collagénique bicomposite selon l'une quelconque des revendications 1 à 14, **caractérisé en ce que** le composant polymère de la couche poreuse formant compresse est choisi parmi les polysaccharides, notamment la chitine ou le chitosane.

17. Matériau collagénique bicomposite selon l'une quelconque des revendications 1 à 14, **caractérisé en ce que** le composant polymère est choisi parmi les polysaccharides modifiés par oxydation des fonctions alcools en fonctions carboxyliques, notamment la cellulose oxydée.

18. Matériau collagénique bicomposite selon l'une quelconque des revendications 1 à 17, **caractérisé en ce que** la couche poreuse formant compresse présente une densité inférieure ou égale à 75 mg/cm², de préférence inférieure à 20 mg/cm².

19. Matériau collagénique bicomposite selon l'une quelconque des revendications 1 à 18, **caractérisé en ce que** la taille des pores de la couche formant compresse varie de 20 à 300 µm.

20. Matériau collagénique bicomposite selon l'une quelconque des revendications 1 à 18, **caractérisé en ce que** la taille moyenne des pores de la couche formant compresse est comprise entre 100 et 200 µm.

21. Matériau collagénique bicomposite selon l'une quelconque des revendications 1 à 20, **caractérisé en ce que** la couche formant compresse présente une épaisseur au moins égale à 0,2 cm et de préférence comprise entre 0,3 cm et 1,5 cm.

22. Procédé pour l'obtention d'un matériau collagénique bicomposite selon l'une quelconque des revendications 1 à 21, **caractérisé en ce que** l'on coule, sur un support inerte convenable, une solution de composant collagénique suivant une épaisseur destinée à la formation d'un film, et **en ce que** l'on applique sur ladite solution en cours de gélification, une compresse non tassée de composant polymère, puis **en ce que** l'on sèche ou laisse sécher le matériau obtenu.

23. Procédé selon la revendication 22, **caractérisé en ce que**, pour la formation du film, on prépare une solution de composant collagénique dont la concentration en collagène est comprise entre 5 et 50 g/l et est de préférence de 30 g/l.

24. Procédé selon la revendication 23, **caractérisé en ce que** l'on prépare une solution acide de collagène natif.

25. Procédé selon l'une quelconque des revendications 22 à 24, **caractérisé en ce que** le composant collagénique consiste en ou comprend du collagène modifié par coupure oxydative.

26. Procédé selon la revendication 25, **caractérisé en ce que** le collagène est modifié par traitement à l'acide periodique ou l'un de ses sels.

27. Procédé selon l'une quelconque des revendications 22 à 26, **caractérisé en ce que** la solution de composant collagénique est chauffée à une température supérieure à 37°C, de préférence à une température comprise entre 40 et 50°C.

28. Procédé selon l'une quelconque des revendications 22 à 27, **caractérisé en ce qu'**on ajoute à la solution de composant collagénique, au moins un additif hydrophile macromoléculaire chimiquement non réactif vis-à-vis du composant collagénique tel que défini selon l'une quelconque des revendications 7 à 11.

29. Procédé selon la revendication 28, **caractérisé en ce que** la concentration en additif(s) hydrophile(s) est 2 à 10 fois inférieure à la concentration en composant collagénique.

30. Procédé selon l'une quelconque des revendications 28 et 29, **caractérisé en ce qu'**on ajoute de la glycérine à la solution de composant collagénique.

31. Procédé selon la revendication 30, **caractérisé en ce que** la concentration en glycérine est comprise entre 3 et 8 g/l et ne dépasse pas le tiers de la concentration en composant collagénique.

32. Procédé selon l'une quelconque des revendications 22 à 31, **caractérisé en ce que**, pour la formation du film, on prépare une solution aqueuse contenant 2 à 10 % de composant collagénique, 0,6 à 4 % d'additif(s) hydrophile(s) et 0,3 à 2,5 % de glycérine s'ils sont présents.

33. Procédé selon l'une quelconque des revendications 22 à 32, **caractérisé en ce que** la solution destinée à la formation du film est neutralisée.

34. Procédé selon l'une quelconque des revendications 22 à 33, **caractérisé en ce que** le support est un support en PVC ou polystyrène.

35. Procédé selon l'une quelconque des revendications 22 à 34, **caractérisé en ce que** la solution destinée à la formation du film, qui est coulée sur le support, présente une densité comprise entre 0,1 et 0,3 g/cm².

36. Procédé selon l'une quelconque des revendications 22 à 35, **caractérisé en ce que** le coulage de la solution collagénique est réalisé à une température comprise entre 4 et 30°C, de préférence entre 18 et 25°C.

37. Procédé selon l'une quelconque des revendications 22 à 36, **caractérisé en ce que** l'on utilise une compresse à base de collagène.

38. Procédé selon la revendication 37, **caractérisé en ce que** la compresse poreuse est préparée à partir d'une solution aqueuse acide de collagène dont la concentration est comprise entre 2 et 50 g/l, et est de préférence de 10 g/l lorsque le collagène est non dénaturé.

39. Procédé selon la revendication 38, **caractérisé en ce que** la solution est neutralisée jusqu'à un pH de l'ordre de 7 à 8.

40. Procédé selon l'une quelconque des revendications 38 et 39, **caractérisé en ce que** la solution est lyophilisée.

41. Procédé selon la revendication 40, **caractérisé en ce que** la solution est répartie en couche présentant une densité comprise entre 0,2 et 1,5 g/cm² pour la lyophilisation.

42. Procédé selon l'une quelconque des revendications 22 à 36, **caractérisé en ce que** l'on utilise une compresse hémostatique à base de polysaccharide, notamment de chitine ou de chitosane.

43. Procédé selon l'une quelconque des revendications 22 à 36, **caractérisé en ce que** l'on utilise une compresse hémostatique à base de polysaccharide modifié par oxydation des fonctions alcools en fonctions carboxyliques, notamment de cellulose oxydée.

44. Procédé selon l'une quelconque des revendications 22 à 43, **caractérisé en ce que** lors de l'application de la compresse poreuse sur la solution destinée à la formation du film en cours de gélification, on laisse pénétrer la compresse sur une distance de l'ordre de 0,05 à 2 mm, de préférence de l'ordre de 0,1 à 0,5 mm dans le gel en cours de formation.

45. Procédé selon l'une quelconque des revendications 22 à 44, **caractérisé en ce que** l'on sèche le matériau obtenu dans un flux d'air stérile.

46. Procédé selon l'une quelconque des revendications 22 à 45, **caractérisé en ce que** la compresse poreuse est réalisée par lyophilisation d'une émulsion collagénique et d'un gaz.

47. Procédé selon l'une quelconque des revendications 22 à 46, **caractérisé en ce que** le matériau obtenu est stérilisé, notamment par irradiation avec des rayonnements bêta ou gamma.

## Claims

1. Bicomposite collagenic material which is biocompatible, non-toxic and biodegradable in less than a month, **characterized in that** it comprises only or mainly two closely bound layers, these being a layer forming a film based on a collagenic constituent, notably collagen which has at least partially lost its helical structure, or gelatine, and a layer forming a porous compress, non compacted based on a polymer constituent.

2. Bicomposite collagenic material according to claim 1, **characterized in that** the collagenic constituent consists of or includes collagen which has at least partially lost its helical structure.

3. Bicomposite collagenic material according to claim 2, **characterized in that** the collagenic constituent is formed of non-hydrolyzed collagen, consisting mainly of α chains.

4. Bicomposite collagenic material according to any of claims 1 to 3, **characterized in that** the collagenic constituent consists of or includes gelatine.

5. Collagenic material according to any of claims 1 to 4, **characterized in that** the collagenic constituent consists of or includes collagen modified by oxidative cleavage.

6. Bicomposite collagenic material according to any of claims 1 to 5, **characterized in that** the layer forming the film also includes at least one macromolecular hydrophilic additive which does not react chemically with the collagenic constituent.

7. Bicomposite collagenic material according to claim 6, **characterized in that** the macromolecular hydrophilic additive has a molecular weight of more than 3000 Daltons.

8. Bicomposite collagenic material according to claim 7, **characterized in that** the macromolecular hydrophilic additive has a molecular weight of between 3,000 and 20,000 Daltons.

9. Bicomposite collagenic material according to any of claims 6 to 8, **characterized in that** the macromolecular hydrophilic additive is polyethylene glycol.

10. Bicomposite collagenic material according to any of claims 6 to 9, **characterized in that** the hydrophilic additive is chosen from polysaccharides, notably starch, dextran or cellulose, or mucopolysaccharides.

11. Bicomposite collagenic material according to any of claims 6 to 10, **characterized in that** the hydrophilic additive is an oxidized polysaccharide.

12. Bicomposite collagenic material according to any of claims 6 to 11, **characterized in that** the concentration of hydrophilic additive(s) is 2 to 10 times less than that of the collagenic constituent.

13. Bicomposite collagenic material according to any of claims 6 to 12, **characterized in that** the layer forming the film also includes glycerine.

14. Bicomposite collagenic material according to any of claims 1 to 13, **characterized in that** the layer forming the film is less than 100 µm thick, and preferably between 30 µm and 75 µm.

15. Bicomposite collagenic material according to any of claims 1 to 14, **characterized in that** the polymer constituent of the porous layer forming the compress consists of or includes collagen.

16. Bicomposite collagenic material according to any of claims 1 to 14, **characterized in that** the polymer constituent of the porous layer forming the compress is chosen from the polysaccharides, notably chitin or chitosan.

17. Bicomposite collagenic material according to any of claims 1 to 14, **characterized in that** the polymer constituent is chosen from polysaccharides modified by oxidation of the alcohol functions into carboxylic functions, notably oxidized cellulose.

18. Bicomposite collagenic material according to any of claims 1 to 17, **characterized in that** the porous layer forming the compress has a density of not more than 75 mg/cm² and preferably less than 20 mg/cm².

19. Bicomposite collagenic material according to any of claims 1 to 18, **characterized in that** the size of the pores in the layer forming the compress vary from 20 to 300 µm.

20. Bicomposite collagenic material according to any of claims 1 to 18, **characterized in that** the average size of the pores in the layer forming the compress is between 100 and 200 µm.

21. Bicomposite collagenic material according to any of claims 1 to 20, **characterized in that** the layer forming the compress is at least 0.2 cm thick and preferably between 0.3 cm and 1.5 cm.

22. Process to obtain a bicomposite collagenic material according to any of claims 1 to 21, **characterized in that** a solution of collagenic constituent is poured onto an appropriate inert support to a thickness suitable for forming a film, and **in that** a substantially non compacted compress made of a polymer constituent is applied to the said solution during the gelling process and **in that** the material obtained is dried or left to dry.

23. Process according to claim 22, **characterized in that**, for the formation of the film, a solution of the collagenic constituent is prepared in which the concentration of collagen is between 5 and 50 g/l and preferably 30 g/l.

24. Process according to claim 23, **characterized in that** an acid solution of native collagen is prepared.

25. Process according to any of claims 22 to 24, **characterized in that** the collagenic constituent consists of or includes collagen modified by oxidative cleavage.

26. Process according to claim 25, **characterized in that** the collagen is modified by treatment with periodic acid or one of its salts.

27. Process according to any of claims 22 to 26, **characterized in that** the solution of collagenic constituent is heated to a temperature of more than 37°C and preferably a temperature of between 40 and 50°C.

28. Process according to any of claims 22 to 27, **characterized in that** at least one macromolecular hydrophilic additive, chemically unreactive with respect to the collagenic constituent as defined in any of claims 7 to 11 is added to the solution of collagenic constituent.

29. Process according to claim 28, **characterized in that** the concentration of hydrophilic additive(s) is 2 to 10 times less than the concentration of the collagenic constituent.

30. Process according to either of claims 28 and 29, **characterized in that** glycerine is added to the solution of collagenic constituent.

31. Process according to claim 30, **characterized in that** the concentration of glycerine is between 3 and 8 g/l and does not exceed one third of the concentration of the collagenic constituent.

32. Process according to any of claims 22 to 31, **characterized in that**, for formation of the film, an aqueous solution containing 2 to 10% of collagenic constituent, 0.6 to 4% of hydrophilic additive(s) and 0.3 to 2.5% of glycerine if they are present, is prepared.

33. Process according to any of claims 22 to 32, **characterized in that** the solution destined to form the film is neutralized.

34. Process according to any of claims 22 to 33, **characterized in that** the support is a PVC or polystyrene support.

35. Process according to any of claims 22 to 34, **characterized in that** the solution destined to form the film, which is poured onto the support, has a density of between 0.1 and 0.3 g/cm².

36. Process according to any of claims 22 to 35, **characterized in that** the collagenic solution is poured at a temperature of 4 to 30°C, preferably between 18 and 25°C.

37. Process according to any of claims 22 to 36, **characterized in that** a collagen based compress is used.

38. Process according to claim 37, **characterized in that** the porous compress is prepared from an aqueous acid solution of collagen, the concentration of which is 2 to 50 g/l and preferably 10 g/l when the collagen is not denatured.

39. Process according to claim 38, **characterized in that** the solution is neutralized to a pH of around 7 to 8.

40. Process according to either of claims 38 or 39, **characterized in that** the solution is freeze-dried.

41. Process according to claim 40, **characterized in that** the solution is spread in a layer with a density of between 0.2 and 1.5 g/cm² for freeze-drying.

42. Process according to any of claims 22 to 36, **characterized in that** a hemostatic compress based on polysaccharide, notably chitin or chitosan is used.

43. Process according to any of claims 22 to 36, **characterized in that** a hemostatic compress based on polysaccharide modified by oxidation of the alcohol functions into carboxylic function, notably oxidized cellulose is used.

44. Process according to any of claims 22 to 43, **characterized in that** when the porous compress is applied to the solution destined to form the film during gelling, the compress is allowed to penetrate for around 0.05 to 2 mm, preferably around 0.1 to 0.5 mm in the gel which is forming.

45. Process according to any of claims 22 to 44, **characterized in that** the material obtained is dried in a jet of sterile air.

46. Process according to any of claims 22 to 45, **characterized in that** the porous compress is produced by freeze-drying a collagenic emulsion and a gas.

47. Process according to any of claims 22 to 46, **characterized in that** the material obtained is sterilized, notably by beta or gamma irradiation.

## Patentansprüche

1. Biologisch verträgliches, nichttoxisches und in mindestens einem Monat biologisch abbaubares Zweikomponenten-Collagen-Material, **dadurch gekennzeichnet, daß** es ausschließlich oder überwiegend zwei innig verbundene Schichten umfaßt, das heißt eine Schicht, die einen Film auf der Grundlage einer Collagen-Komponente, insbesondere Collagen, welches mindestens teilweise seine Helix-Struktur verloren hat, oder Gelatine, bildet, und eine Schicht, die eine nicht zusammengepreßte, poröse Kompresse bildet, auf der Grundlage einer Polymer-Komponente.

2. Zweikomponenten-Collagen-Material nach Anspruch 1, **dadurch gekennzeichnet, daß** die Collagen-Komponente aus einem Collagen, welches mindestens teilweise seine Helix-Struktur verloren hat, besteht oder ein solches enthält.

3. Zweikomponenten-Collagen-Material nach Anspruch 2, **dadurch gekennzeichnet, daß** die Collagen-Komponente aus nichthydrolysiertem Collagen gebildet ist, welches überwiegend aus α-Ketten aufgebaut ist.

4. Zweikomponenten-Collagen-Material nach einem der Ansprüche 1 bis 3, **dadurch gekennzeichnet, daß** die Collagen-Komponente aus Gelatine besteht oder diese enthält.

5. Collagen-Material nach einem der Ansprüche 1 bis 4, **dadurch gekennzeichnet, daß** die Collagen-Komponente aus durch oxidative Spaltung modifiziertem Collagen besteht oder dieses enthält.

6. Zweikomponenten-Collagen-Material nach einem der Ansprüche 1 bis 5, **dadurch gekennzeichnet, daß** die den Film bildende Schicht zusätzlich mindestens ein chemisch gegenüber der Collagen-Komponente nicht reaktives, makromolekulares, hydrophiles Additiv enthält.

7. Collagen-Material nach Anspruch 6, **dadurch gekennzeichnet, daß** das makromolekulare, hydrophile Additiv ein Molekulargewicht von mehr als 3000 Dalton aufweist.

8. Zweikomponenten-Collagen-Material nach Anspruch 7, **dadurch gekennzeichnet, daß** das makromolekulare, hydrophile Additiv ein Molekulargewicht zwischen 3 000 und 20 000 Dalton aufweist.

9. Zweikomponenten-Collagen-Material nach einem der Ansprüche 6 bis 8, **dadurch gekennzeichnet, daß** das makromolekulare, hydrophile Additiv Polyethylenglykol ist.

10. Zweikomponenten-Collagen-Material nach einem der Ansprüche 6 bis 9, **dadurch gekennzeichnet, daß** das hydrophile Additiv aus Polysacchariden, insbesondere Stärke, Dextran oder Cellulose, oder Mucopolysacchariden ausgewählt ist.

11. Zweikomponenten-Collagen-Material nach einem der Ansprüche 6 bis 10, **dadurch gekennzeichnet, daß** das hydrophile Additiv ein Polysaccharid in oxidierter Form ist.

12. Zweikomponenten-Collagen-Material nach einem der Ansprüche 6 bis 11, **dadurch gekennzeichnet, daß** die Konzentration des (der) hydrophilen Additivs(e) 2- bis 10-mal geringer ist als die der Collagen-Komponente.

13. Zweikomponenten-Collagen-Material nach einem der Ansprüche 6 bis 12, **dadurch gekennzeichnet, daß** die den Film bildende Schicht zusätzlich Glycerin enthält.

14. Zweikomponenten-Collagen-Material nach einem der Ansprüche 1 bis 13, **dadurch gekennzeichnet, daß** die den Film bildende Schicht eine Dicke von weniger als 100 µm, vorzugsweise zwischen 30 µm und 75 µm, aufweist.

15. Zweikomponenten-Collagen-Material nach einem der Ansprüche 1 bis 14, **dadurch gekennzeichnet, daß** die Polymer-Komponente der die Kompresse bildenden porösen Schicht aus Collagen besteht oder dieses enthält.

16. Zweikomponenten-Collagen-Material nach einem der Ansprüche 1 bis 14, **dadurch gekennzeichnet, daß** die Polymer-Komponente der die Kompresse bildenden porösen Schicht aus Polysacchariden, insbesondere Chitin und Chitosan, ausgewählt ist.

17. Zweikomponenten-Collagen-Material nach einem der Ansprüche 1 bis 14, **dadurch gekennzeichnet, daß** die Polymer-Komponente aus Polysacchariden, die durch Oxidation der Alkoholfunktionen zu Carboxylfunktionen modifiziert worden sind, insbesondere oxidierter Cellulose, ausgewählt ist.

18. Zweikomponenten-Collagen-Material nach einem der Ansprüche 1 bis 17, **dadurch gekennzeichnet, daß** die die Kompresse bildende poröse Schicht eine Dichte von 75 mg/cm² oder weniger, vorzugsweise weniger als 20 mg/cm², aufweist.

19. Zweikomponenten-Collagen-Material nach einem der Ansprüche 1 bis 18, **dadurch gekennzeichnet, daß** die Porengröße der die Kompresse bildenden Schicht von 20 bis 300 µm variiert.

20. Zweikomponenten-Collagen-Material nach einem der Ansprüche 1 bis 18, **dadurch gekennzeichnet, daß** die durchschnittliche Porengröße der die Kompresse bildenden Schicht zwischen 100 und 200 µm liegt.

21. Zweikomponenten-Collagen-Material nach einem der Ansprüche 1 bis 20, **dadurch gekennzeichnet, daß** die die Kompresse bildende Schicht eine Dicke von mindestens 0,2 cm, die vorzugsweise zwischen 0,3 und 1,5 cm liegt, aufweist.

22. Verfahren zur Herstellung eines Zweikomponenten-Collagen-Materials nach einem der Ansprüche 1 bis 21, **dadurch gekennzeichnet, daß** man eine Lösung der Collagen-Komponente in einer Dicke, die für die Bildung eines Films bestimmt ist, auf eine geeignete inerte Unterlage gießt und im Verlaufe der Gelbildung auf diese Lösung eine nicht zusammengepreßte Kompresse aus dem Polymer-Bestandteil aufbringt und man dann das erhaltene Material trocknet oder trocknen läßt.

23. Verfahren nach Anspruch 22, **dadurch gekennzeichnet, daß** man zur Bildung des Films eine Lösung der Collagen-Komponente bereitet, deren Collagen-Konzentration zwischen 5 und 50 g/l liegt und vorzugsweise 30 g/l beträgt.

24. Verfahren nach Anspruch 23, **dadurch gekennzeichnet, daß** man eine saure Lösung von nativem Collagen bereitet.

25. Verfahren nach einem der Ansprüche 22 bis 24, **dadurch gekennzeichnet, daß** die Collagen-Komponente aus durch oxidative Spaltung modifiziertem Collagen besteht oder dieses enthält.

26. Verfahren nach Anspruch 25, **dadurch gekennzeichnet, daß** das Collagen durch Behandeln mit Periodsäure oder einem ihrer Salze modifiziert wird.

27. Verfahren nach einem der Ansprüche 22 bis 26, **dadurch gekennzeichnet, daß** die Lösung der Collagen-Komponente auf eine Temperatur von mehr als 37°C, vorzugsweise auf eine Temperatur zwischen 40 und 50°C erhitzt wird.

28. Verfahren nach einem der Ansprüche 22 bis 27, **dadurch gekennzeichnet, daß** man zu der Lösung der Collagen-Komponente mindestens ein gegenüber der Collagen-Komponente chemisch nicht reaktives, makromolekulares, hydrophiles Additiv zusetzt, wie es in einem der Ansprüche 7 bis 11 definiert ist.

29. Verfahren nach Anspruch 28, **dadurch gekennzeichnet, daß** die Konzentration des (der) hydrophilen Additiv(e) 2- bis 10-mal geringer ist als die Konzentration der Collagen-Komponente.

30. Verfahren nach einem der Ansprüche 28 und 29, **dadurch gekennzeichnet, daß** man Glycerin zu der Lösung der Collagen-Komponente zugibt.

31. Verfahren nach Anspruch 30, **dadurch gekennzeichnet, daß** die Glycerin-Konzentration zwischen 3 und 8 g/l liegt und nicht ein Drittel der Konzentration der Collagen-Komponente übersteigt.

32. Verfahren nach einem der Ansprüche 22 bis 31, **dadurch gekennzeichnet, daß** man zur Bildung des Films eine wäßrige Lösung bereitet, die 2 bis 10 % der Collagen-Komponente und, falls vorhanden, 0,6 bis 4 % des (der) hydrophilen Additiv(e) und 0,3 bis 2,5 % Glycerin enthält.

33. Verfahren nach einem der Ansprüche 22 bis 32, **dadurch gekennzeichnet, daß** die zur Bildung des Films bestimmte Lösung neutralisiert wird.

34. Verfahren nach einem der Ansprüche 22 bis 33, **dadurch gekennzeichnet, daß** die Unterlage eine Unterlage aus PVC oder Polystyrol ist.

35. Verfahren nach einem der Ansprüche 22 bis 34, **dadurch gekennzeichnet, daß** die zur Bildung des Films bestimmte Lösung, die auf die Unterlage vergossen wird, eine Dichte zwischen 0,1 und 0,3 g/cm² aufweist.

36. Verfahren nach einem der Ansprüche 22 bis 35, **dadurch gekennzeichnet, daß** das Aufgießen der Collagen-Lösung bei einer Temperatur zwischen 4 und 30°C, vorzugsweise zwischen 18 und 25°C, erfolgt.

37. Verfahren nach einem der Ansprüche 22 bis 36, **dadurch gekennzeichnet, daß** man eine Kompresse auf der Grundlage von Collagen verwendet.

38. Verfahren nach Anspruch 37, **dadurch gekennzeichnet, daß** die poröse Kompresse ausgehend von einer wäßrigen sauren Collagen-Lösung hergestellt wird, deren Konzentration zwischen 2 und 50 g/l liegt und vorzugsweise 10 g/l beträgt, wenn das Collagen nicht denaturiert ist.

39. Verfahren nach Anspruch 38, **dadurch gekennzeichnet, daß** die Lösung bis zu einem pH-Wert im Bereich von 7 bis 8 neutralisiert wird.

40. Verfahren nach einem der Ansprüche 38 und 39, **dadurch gekennzeichnet, daß** die Lösung gefriergetrocknet wird.

41. Verfahren nach Anspruch 40, **dadurch gekennzeichnet, daß** die Lösung für die Gefriertrocknung zu einer Schicht mit einer Dichte zwischen 0.2 und 1,5 g/cm² verteilt wird.

42. Verfahren nach einem der Ansprüche 22 bis 36, **dadurch gekennzeichnet, daß** man eine hämostatische Kompresse auf der Grundlage eines Polysaccharids, insbesondere Chitin oder Chitosan, verwendet.

43. Verfahren nach einem der Ansprüche 22 bis 36, **dadurch gekennzeichnet, daß** man eine hämostatische Kompresse auf der Grundlage von durch Oxidation der Alkoholfunktionen zu Carboxylfunktionen modifiziertem Polysaccharid, insbesondere oxidierter Cellulose, verwendet.

44. Verfahren nach einem der Ansprüche 22 bis 43, **dadurch gekennzeichnet, daß** man beim Aufbringen der porösen Kompresse im Verlaufe der Gelbildung auf die zur Bildung des Films bestimmte Lösung die Kompresse bis zu einer Tiefe im Bereich von 0,05 bis 2 mm, vorzugsweise im Bereich von 0.1 bis 0.5 mm, in das sich bildende Gel eindringen läßt.

45. Verfahren nach einem der Ansprüche 22 bis 44, **dadurch gekennzeichnet, daß** man das erhaltene Material in einem sterilen Luftstrom trocknet.

46. Verfahren nach einem der Ansprüche 22 bis 45, **dadurch gekennzeichnet, daß** die poröse Kompresse durch Gefriertrocknen einer Collagen-Emulsion und eines Gases gebildet wird.

47. Verfahren nach einem der Ansprüche 22 bis 46, **dadurch gekennzeichnet, daß** das erhaltene Material insbesondere durch Bestrahlung mit beta- oder gamma-Strahlen sterilisiert wird.
